# EUROPEAN PATENT SPECIFICATION

(11) **EP 4 541 276 B1**
(45) Date of publication and mention of the grant of the patent: **16.09.2026**
(21) Application number: 24186554.2
(22) Date of filing: 04.07.2024
(51) Int. Cl.: A61B 5/145, A61B 5/1455, A61B 5/00

(54) **NON-INVASIVE BLOOD GLUCOSE MONITORING DEVICE WITH WEARING FIT DETECTION FUNCTION**
NICHTINVASIVE BLUTZUCKERÜBERWACHUNGSVORRICHTUNG MIT TRAGEERKENNUNGSFUNKTION
DISPOSITIF DE SURVEILLANCE NON INVASIVE DE LA GLYCÉMIE AVEC FONCTION DE DÉTECTION D'AJUSTEMENT AU PORT

(30) Priority: 20.10.2023 TW 112140307
(43) Date of publication of application: 23.04.2025
(73) Proprietor: Taiwan-Asia Semiconductor Corporation, Hsinchu City 30078 (TW)
(72) Inventor: Dai, Shu-Wen, 30078 Hsinchu City (TW); Chen, Sheng-Wei, 30078 Hsinchu City (TW)
(74) Representative: Michalski Hüttermann & Partner mbB

(56) References cited:
- US-A1- 2017 296 124
- US-A1- 2021 290 120
- US-A1- 2023 010 168
- US-B2- 11 160 504

## Description

### BACKGROUND OF THE INVENTION

### 1. Field of the Invention

The present invention relates to a blood glucose monitoring device, especially a non-invasive blood glucose monitoring device with a wearing fit detection function.

### 2. Description of Related Art

Most conventional non-invasive blood glucose monitoring devices are worn on specific parts of bodies of users, such as upper limbs or lower limbs. Through detection units, attached to skin surfaces of the specific parts, sending and receiving of optical signals are performed, to detect blood glucose values in the blood of the users. Conventional non-invasive blood glucose monitoring devices must be fixed on the specific parts of the bodies of the users through accessories such as bracelets, fixing straps, adhesives, etc.. For the users, once wearing is incorrectly operated or positioning is deviated, the conventional non-invasive blood glucose monitoring devices cannot adhere closely to the skins, resulting in larger gaps therebetween, which easily causes the optical signals lost during sending or receiving processes and thus affects detection accuracy.

US 2017/296124 A1 describes a wearable sensor device which comprises a heart rate monitor, a motion sensor and an optical sensor.

US 2023/010168 A1 describes a wearable electronic device comprises a housing, a first electrode and a second electrode disposed on the housing, an A/D converter connected to the first electrode and the second electrode, a pulse generator connected to the first electrode and the second electrode and a processor operatively connected to the A/D converter and the pulse generator.

US 2021/290120 A1 describes a wearable health monitoring device which can include a physiological parameter measurement sensor or module configured to be in contact with a wearer's skin when the device is worn by the wearer on the wrist.

US 11 160 504 B2 describes an electronic device including a plurality of electrodes selectively connectable to a touch sensor or one or more biometric sensors and a processor configured to receive a user input through the plurality of electrodes in a state in which the touch sensor and the plurality of electrodes are connected.

In light of this, it is really worthy of research and development, for solving those above-mentioned problems, to design a non-invasive blood glucose monitoring device.

### SUMMARY OF THE INVENTION

An objective of the present invention is to provide a non-invasive blood glucose monitoring device with a wearing fit detection function.

The invention is defined by the features of the independent claim.

To achieve the above mentioned objectives, the non-invasive blood glucose monitoring device with the wearing fit detection function provided by the present invention comprises a substrate, a blood glucose monitoring module, an optical feedback sensing module and at least one light-blocking wall. The substrate defines a first setting area and a second setting area, and the second setting area is between an edge of the substrate and the first setting area. The blood glucose monitoring module is disposed in the first setting area and the optical feedback sensing module is disposed in the second setting area. The optical feedback sensing module includes at least one light-emitting element and at least one light-receiving element. Each light-emitting element emits a light signal corresponding to at least one specific wavelength and each light-receiving element receives the light signal corresponding to the at least one specific wavelength, after being reflected, as a basis to determine a wearing fit. The at least one light-blocking wall is disposed on the substrate and located between the first setting area and the second setting area to block the blood glucose monitoring module and the optical feedback sensing module.

In one embodiment of the present invention, each light-emitting element is a multi-chip LED which correspondingly emits a plurality of light signals of specific wavelengths.

In one embodiment of the present invention, the light-receiving element corresponds to a wavelength response range, and the plurality of specific wavelengths fall within the wavelength response range.

In one embodiment of the present invention, the wavelength response range is between 500 nm and 1100 nm.

In one embodiment of the present invention, a center distance between each light-emitting element and its adjacent light-receiving element is between 1 mm and 4 mm.

In one embodiment of the present invention, a quantity of the at least one light-emitting element is not less than a quantity of the at least one light-receiving element.

In one embodiment of the present invention, when the at least one light-emitting element and the at least one light-receiving element are both plural, the light-emitting elements and the light-receiving elements are arranged symmetrically relative to the blood glucose monitoring module.

In one embodiment of the present invention, the non-invasive blood glucose monitoring device with the wearing fit detection function further comprises a processing unit, which mainly adopts photoplethysmography to analyze the reflected light signal corresponding to the at least one specific wavelength for determining the wearing fit.

In one embodiment of the present invention, the first setting area is located at a center of the substrate, and the second setting area surrounds the first setting area.

In one embodiment of the present invention, the optical feedback sensing module further includes at least one blocking structure, wherein each blocking structure is disposed between any light-emitting element and its adjacent light-receiving element, and wherein each blocking structure extends from the edge of the substrate and connects to or approaches any light-blocking wall.

In one embodiment of the present invention, the non-invasive blood glucose monitoring device with the wearing fit detection function further comprises a packaging structure which is disposed on the substrate and adopted to package the blood glucose monitoring module and the optical feedback sensing module.

In one embodiment of the present invention, the non-invasive blood glucose monitoring device with the wearing fit detection function further comprises a transparent cover which is disposed on the packaging structure to protect the blood glucose monitoring module and the optical feedback sensing module.

Accordingly, for the present invention, by disposing the optical feedback sensing module and by using photoplethysmography method to analyze the light signals cooperatively, whether the user has correctly worn the non-invasive blood glucose monitoring device can be confirmed. Compared with conventional non-invasive blood glucose monitoring devices, reliability and accuracy of blood glucose detection are further ensured. And by arranging at least one light-blocking wall, mutual interferences of signal detecting between the blood glucose monitoring module and the optical feedback sensing module can be avoided.

### BRIEF DESCRIPTION OF THE DRAWINGS

FIG. 1 is a schematic view of a non-invasive blood glucose monitoring device with a wearing fit detection function according to the present invention;
FIG. 2A is a top view of a first embodiment of the non-invasive blood glucose monitoring device with the wearing fit detection function according to the present invention;
FIG. 2B is a cross-sectional view along a line segment A shown in FIG. 2A;
FIG. 2C is a cross-sectional view along a line segment B shown in FIG. 2A;
FIG. 2D is a top view of at least one blocking structure added for an optical feedback sensing module of the first embodiment shown in FIG. 2A;
FIG. 3A is a top view of a second embodiment of the non-invasive blood glucose monitoring device with the wearing fit detection function according to the present invention;
FIG. 3B is a top view of at least one blocking structure added for an optical feedback sensing module of the second embodiment shown in FIG. 3A;
FIG. 4A is a top view of a third embodiment of the non-invasive blood glucose monitoring device with the wearing fit detection function according to the present invention;
FIG. 4B is a top view of at least one blocking structure added for an optical feedback sensing module of the third embodiment shown in FIG. 4A;
FIG. 5A is a top view of a fourth embodiment of the non-invasive blood glucose monitoring device with the wearing fit detection function according to the present invention;
FIG. 5B is a top view of at least one blocking structure added for an optical feedback sensing module of the fourth embodiment shown in FIG. 5A;
FIG. 6A is a top view of a fifth embodiment of the non-invasive blood glucose monitoring device with the wearing fit detection function according to the present invention;
FIG. 6B is a top view of at least one blocking structure added for an optical feedback sensing module of the fifth embodiment shown in FIG. 6A;
FIG. 7A is a top view of a sixth embodiment of the non-invasive blood glucose monitoring device with the wearing fit detection function according to the present invention;
FIG. 7B is a top view of at least one blocking structure added for an optical feedback sensing module of the sixth embodiment shown in FIG. 7A;
FIG. 8A is a top view of a seventh embodiment of the non-invasive blood glucose monitoring device with the wearing fit detection function according to the present invention;
FIG. 8B is a top view of at least one blocking structure added for an optical feedback sensing module of the seventh embodiment shown in FIG. 8A.

### DETAILED DESCRIPTION OF THE PREFERRED EMBODIMENT

Since various examples and embodiments in the present invention are only illustrative and non-restrictive, a person skilled in the art can easily conceive other examples and embodiments without contravening the scope of the present invention, after reading this specification, and can make the features and advantages of these embodiments more evident based on the following detailed description and claims.

Herein, the description of unit, element and component in the present invention uses "one", "a", or "an". This is for convenience and for offering general meaning of the category of the present invention. Therefore, the description should be understood as including "one", "at least one", and singular and plural forms at the same time unless the context clearly indicates otherwise.

Herein, the description of the terms "first" or "second" and similar ordinal numbers are mainly used to distinguish or refer to the same or similar elements or structures and do not necessarily imply that such components or structures are spatially or temporally distinct order. It should be understood that ordinal numbers, in certain situations or configurations, may be used interchangeably without affecting the implementation of the present invention. Herein, the description of "comprise", "have" or other similar semantics have the non-exclusive meaning. For example, components or structures with a plurality of elements are not only limited to those disclosed in this specification, but also include generally inherent elements, which are not explicitly listed here for the components or the structures.

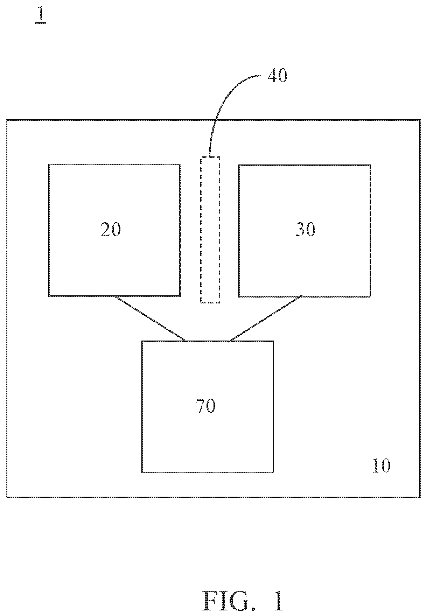

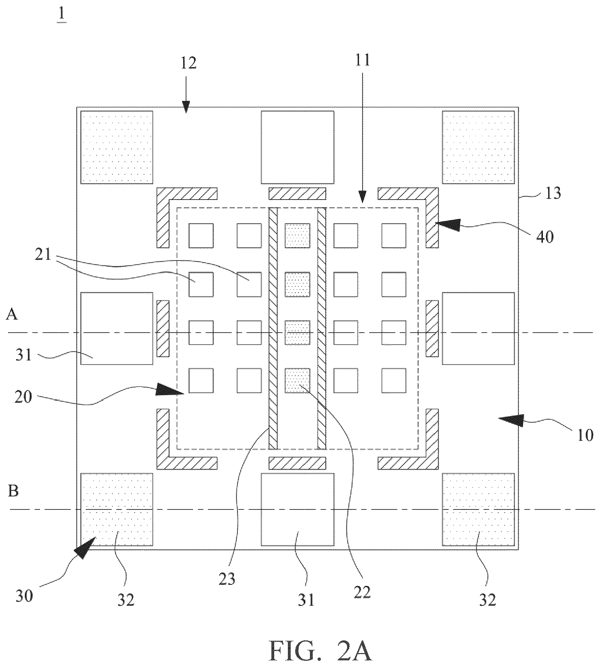

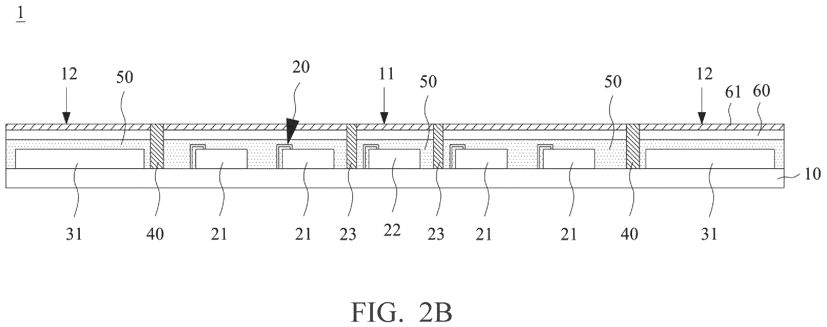

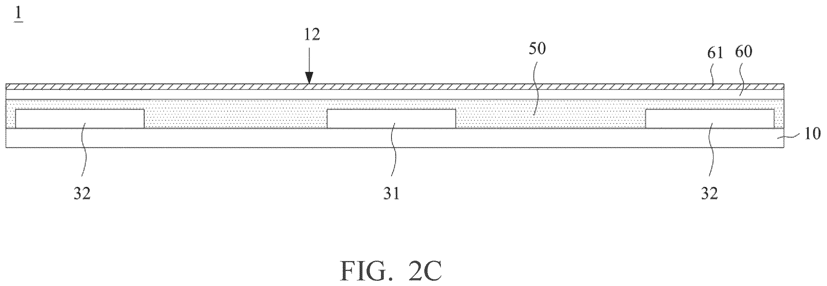

Please refer to FIG. 1 to FIG. 2C together, wherein FIG. 1 is a schematic view of a non-invasive blood glucose monitoring device with a wearing fit detection function according to the present invention, FIG. 2A is a top view of a first embodiment of the non-invasive blood glucose monitoring device with the wearing fit detection function according to the present invention, FIG. 2B is a cross-sectional view along a line segment A shown in FIG. 2A, and FIG. 2C is a cross-sectional view along a line segment B shown in FIG. 2A. As shown in FIG. 1 and FIG. 2A, the non-invasive blood glucose monitoring device with the wearing fit detection function 1 according to the present invention comprises a substrate 10, a blood glucose monitoring module 20, an optical feedback sensing module 30 and at least one light-blocking wall 40. And the blood glucose monitoring module 20, the optical feedback sensing module 30 and the at least one light-blocking wall 40 are disposed on the substrate 10. Conductive circuits are pre-disposed on the substrate 10 for electrically connecting with the blood glucose monitoring module 20 and the optical feedback sensing module 30. In the present invention, the substrate 10 is a ceramic substrate, a silicon substrate or a PCB substrate, but not limited thereto, and the substrate 10 can be made of other materials. An overall structural appearance of the substrate 10 can be modified according to different design requirements. For example, the substrate 10 is rectangular or circular.

In the present invention, a first setting area 11 and a second setting area 12 are defined on the substrate 10, and the second setting area 12 is between an edge 13 of the substrate 10 and the first setting area 11. The first setting area 11 is adopted for setting the blood glucose monitoring module 20, and the second setting area 12 is adopted for setting the optical feedback sensing module 30. As a position of the first setting area 11 is different, a position of the second setting area 12 will also change accordingly. For example, in this embodiment, if the first setting area 11 is located at a center of the substrate 10, the second setting area 12 surrounds the first setting area 11, so that the second setting area 12 is similar to a frame-shaped area surrounding the first setting area 11. But the positions of the first setting area 11 and the second setting area 12 are not limited thereto.

The blood glucose monitoring module 20 is disposed in the first setting area 11 of the substrate 10, and blood glucose states of users are detected by the blood glucose monitoring module 20. The blood glucose monitoring module 20 includes at least one light source 21, at least one photo detector 22 and at least one blocking element 23. Each light source 21 mainly emits light with a fixed wavelength to skins of the users. A single LED can be used for each light source 21, but the type of the light source 21 is not limited thereto. Each light source 21 can be electrically connected to the substrate 10 through a bonding wire. Each photo detector 22 mainly receives the light with the fixed wavelength that is diffusely reflected by the skins and then transmits back. Each photo detector 22 can be electrically connected to the substrate 10 through a bonding wire. Each blocking element 23 is utilized to separate at least one light source 21 and its adjacent at least one photo detector 22. Each blocking element 23 can be made of black plastic or other materials with non-transparent (including visible light and invisible light) properties. Quantities and positions of at least one light source 21, of at least one photo detector 22 and of at least one blocking element 23 vary according to different design requirements. For example, in this embodiment, sixteen light sources 21 and four photo detectors 22 are configured to form a 4x5 matrix for the blood glucose monitoring module 20, and the photo detectors 22, by two blocking elements 23 disposed on both sides thereof, are separated from the light sources 21, to perform the blood glucose monitoring function. However, the present invention is not limited thereto.

The optical feedback sensing module 30 is disposed in the second setting area 12 of the substrate 10, and the optical feedback sensing module 30 detects the wearing fit when the user wears the device. The optical feedback sensing module 30 includes at least one light-emitting element 31 and at least one light-receiving element 32. Each light-emitting element 31 emits a light signal corresponding to at least one specific wavelength. In the present invention, a multi-chip LED light source is adopted for each light-emitting element 31, which correspondingly emits a plurality of light signals of specific wavelengths through a plurality of LED chips contained therein. However, according to different design requirements, a single LED light source can be adopted for each light-emitting element 31 to emit one light signal of one single specific wavelength. A type, a location and a quantity of at least one light-emitting element 31 can be adjusted according to different design requirements. In an embodiment of the present invention, each of the aforementioned specific wavelengths is between 500 nm and 1100 nm. It should be noted that any specific wavelength corresponding to the light signal emitted by each light-emitting element 31 of the optical feedback sensing module 30 is different from the fixed wavelength corresponding to the light signal emitted by each light source 21 of the blood glucose monitoring module 20, so as to avoid mutual interferences between the blood glucose monitoring function and the wearing fit detection function. The light-emitting element 31 can be electrically connected to the substrate 10 through a bonding wire.

Each light-receiving element 32 receives the light signal corresponding to the at least one specific wavelength after being reflected by the skin as a basis to determine the wearing fit. In the present invention, a single photo detector is applied for each light-receiving element 32, and the light-receiving element 32 can be designed to correspond to a wavelength response range, so that the least one of the wavelength corresponding to the light signal, emitted by the light-emitting element 31, falls within the wavelength response range, which makes the light signal with the specific wavelength correspondingly sensed by the light-receiving element 32. A type, a location and a quantity of the at least one light-receiving element 32 can be adjusted according to different design requirements. The light-receiving element 32 can be electrically connected to the substrate 10 through a bonding wire.

In one embodiment of the present invention, it is assumed that each light-emitting element 31 is a multi-chip LED light source with four LED chips, and the specific wavelengths corresponding to the light signals emitted by the light-emitting element 31 are respectively 530 nm, 660 nm, 850 nm and 940 nm. Accordingly, each light-receiving element 32 are designed to correspond to a wavelength response range between 500 nm and 1100 nm, so that the plurality of specific wavelengths corresponding to the light signals emitted by the light-emitting element 31 fall within the wavelength response range of the light-receiving element 32.

In one embodiment of the present invention, a quantity of at least one light-emitting element 31 is not less than a quantity of at least one light-receiving element 32. That is to say, in design, each light-receiving element 32 will correspondingly detect the light signals emitted by one or more light-emitting elements 31. For example, one light-receiving element 32 and one light-emitting element 31 are coupled as one independent detection unit for the optical feedback sensing module 30 basically, to perform detecting of the wearing fit of the device. But depending on different design requirements, one light-receiving element 32 is able to match two light-emitting elements 31 as one independent detection unit for the optical feedback sensing module 30. When one light-receiving element 32 matches two light-emitting elements 31, the two light-emitting elements 31 are located on different sides of the light-receiving element 32, and a center distance between each light-emitting element 31 and the light-receiving element 32 is the same. In order to facilitate transmitting and receiving of optical signals for maintaining detection accuracy, in one embodiment of the present invention, the center distance between each light-emitting element 31 and the adjacent light-receiving element 32 is between 1 mm and 4 mm. However, the present invention is not limited thereto.

In one embodiment of the present invention, when the at least one light-emitting element 31 and the at least one light-receiving element 32 are both plural, the light-emitting elements 31 and the light-receiving elements 32 are arranged symmetrically relative to the blood glucose monitoring module 20. For example, in this embodiment, the substrate 10 is structurally square, the first setting area 11 is located in the center of the substrate 10 for setting the blood glucose monitoring module 20, and the second setting area 12 is a rectangular frame area surrounding the first setting area 11 for setting the optical feedback sensing module 30. The optical feedback sensing module 30 has four light-receiving elements 32 and four light-emitting elements 31. The four light-receiving elements 32 are respectively located at four corners of the rectangular frame area of the second setting area 12, and the four light-emitting elements 31 are, relative to the blood glucose monitoring module 20, respectively located in a middle position of two adjacent light-receiving elements 32, so that the light-emitting elements 31 and the light-receiving elements 32 are arranged symmetrically relative to the blood glucose monitoring module 20.

The at least one light-blocking wall 40 is located between the first setting area 11 and the second setting area 12 of the substrate 10 to block the blood glucose monitoring module 20 and the optical feedback sensing module 30. In the present invention, the at least one light-blocking wall 40 can be a plurality of light-blocking walls 40 that are structurally discontinuous and segmentally arranged, and each light-blocking wall 40 is located between any of the light sources 21 and any of the photo detectors 22 of the blood glucose monitoring module 20 or between any of the light-emitting elements 31 and any of the light-receiving elements 32 of the optical feedback sensing module 30. Nevertheless, the at least one light-blocking wall 40 can also be a single light-blocking wall 40 with a continuous structure and an integral shape. Each light-blocking wall 40 can be made of black plastic or other materials with non-transparent (including visible light and invisible light) properties. Accordingly, light signal interferences between the blood glucose monitoring module 20 and the optical feedback sensing module 30 can be prevented by the at least one light-blocking wall 40.

As shown in FIG. 1 to FIG. 2C, the non-invasive blood glucose monitoring device with the wearing fit detection function 1 of the present invention further comprises a packaging structure 50. The packaging structure 50 is disposed on the substrate 10, and the packaging structure 50 is used for packaging the blood glucose monitoring module 20 and the optical feedback sensing module 30. For the packaging structure 50, a packaging material is applied on the substrate 10 to make the blood glucose monitoring module 20 and the optical feedback sensing module 30 completely covered by the packaging material, and then compression molding is performed relative to the packaging material to form the packaging structure 50 wherein the blood glucose monitoring module 20 and the optical feedback sensing module 30 are embedded. The aforementioned packaging material may be transparent liquid optical glue or epoxy resin, but not limited thereto. In one embodiment of the present invention, a height of the packaging structure 50 is greater than heights of the light sources 21 and of the photo detectors 22 of the blood glucose monitoring module 20, and greater than heights of the light-emitting elements 31 and of the light-receiving elements 32 of the optical feedback sensing module 30, so as to reserve spaces for disposing the corresponding bonding wires.

The non-invasive blood glucose monitoring device with the wearing fit detection function 1 of the present invention further comprises a transparent cover 60. The transparent cover 60 is disposed on the packaging structure 50, and the transparent cover 60 is used for protecting the blood glucose monitoring module 20 and the optical feedback sensing module 30. The transparent cover 60 is made of transparent materials, such as glass, but the invention is not limited thereto. The transparent cover 60 can be fixed on the packaging structure 50 by gluing. Since the packaging material forming the packaging structure 50 itself has an adhesive force, an adhesively fixing effect on the transparent cover 60 occurs by the packaging material when the transparent cover 60 is disposed. The transparent cover 50 further includes a coating 61, and the coating 61 is formed on a side of the transparent cover 60 facing away from the substrate 10. Through the arrangement of the coating 61, the transparent cover 60 is able to provide an effect for allowing light in a specific wavelength band to pass through, and to effectively block stray light in other unnecessary wavelength bands. Since the coating 61 is a structural design often adopted for conventional transparent covers, no further details will be given here.

After the packaging structure 50 and the transparent cover 60 are disposed, by performing a half-cutting operation to form grooves on the packaging structure 50 and the transparent cover 60, by performing a glue dispensing operation to fill the grooves with the black glue material, and till the glue material being solidified, the aforementioned at least one blocking element 23 of blood glucose monitoring module 20 and the at least one light-blocking wall 40 can be formed at desired positions. Since the above-mentioned forming technology of the at least one blocking element 23 and the at least one light-blocking wall 40 is a conventional technology, no further details will be given here.

Furthermore, as shown in FIG. 1 to FIG. 2A, the non-invasive blood glucose monitoring device with the wearing fit detection function 1 of the present invention further comprises a processing unit 70. The processing unit 70 is disposed on the substrate 10 or on another independent substrate, or the processing unit 70 may also be an independent device. The processing unit 70 may be a component or device with signal processing functions, such as a processor, a microcontroller, or a computer. The processing unit 70 is electrically connected to the blood glucose monitoring module 20 to control transmitting of light signals corresponding to each light source 21 and to process the light signals received by each photo detector 22 after being reflected by the skins, so as to analyze blood glucose detection results. The processing unit 70 is also electrically connected to the optical feedback sensing module 30 to control the transmitting of light signals corresponding to each light-emitting element 31 and to process the light signals received by each light-receiving element 32 after being reflected by the skins, so as to analyze detection results of the wearing fit.

In one embodiment of the present invention, the processing unit 70 mainly adopts photoplethysmography (PPG) to analyze each light signal corresponding to at least one specific wavelength reflected by the skin and received by each light-receiving element 32, and determines the wearing fit of the device based on a PPG signal intensity. When it is determined that the PPG signal intensity exceeds a threshold, it means that a better wearing fit occurs around the position of the corresponding light-receiving element 32. On the contrary, when it is determined that the PPG signal intensity does not reach the threshold, it means that a worse wearing fit occurs around the position of thecorresponding light-receiving element 32. When a result of the worse wearing fit is determined, the processing unit 70 will send a notice to an alarm unit (not shown in figures) electrically connected thereto, and the alarm unit delivers sound, light, vibration or other types of warning signals, for reminding the user to re-wear the non-invasive blood glucose monitoring device with the wearing fit detection function 1 of the present invention.

As for the first embodiment of the present invention, the substrate 10 is structurally square, the blood glucose monitoring module 20 is arranged in the first setting area 11 in the center of the substrate 10, and the optical feedback sensing module 30 is arranged in the rectangular frame area of the second setting area 12 on the periphery. For the optical feedback sensing module 30, a detection combination of one light-emitting element 31 and two light-receiving elements 32 forms in each linear portion of the second setting area 12. Accordingly, through the two light-receiving elements 32 provided at any two adjacent corners and the light-emitting element 31 sandwiched therebetween, the wearing fit inside the edge 13 of the substrate 10 corresponding to each linear portion can be detected. Furthermore, through symmetrically arranging of the four light-receiving elements 32 and the four light-emitting elements 31, the wearing fit in whole of the device can be detected.

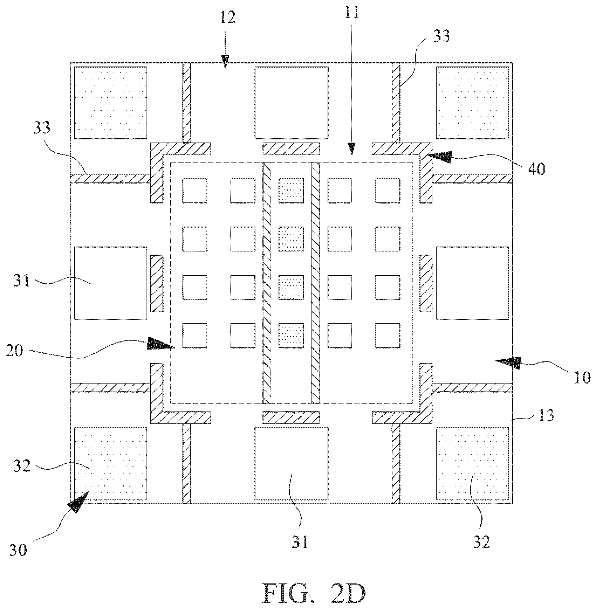

In addition, as shown in FIG. 2D, for the first embodiment of the present invention, the optical feedback sensing module 30 further includes at least one blocking structure 33. Each blocking structure 33 is disposed between any light-emitting element 31 and its adjacent light-receiving element 32 to block the light-emitting element 31 and its adjacent light-receiving element 32. Each blocking structure 33 can extend from the edge 13 of the substrate 10 and connect to any light-blocking wall 40. At least one blocking structure 33 can also be formed by the molding technology similar to that of the aforementioned at least one blocking element 23 and at least one light-blocking wall 40.

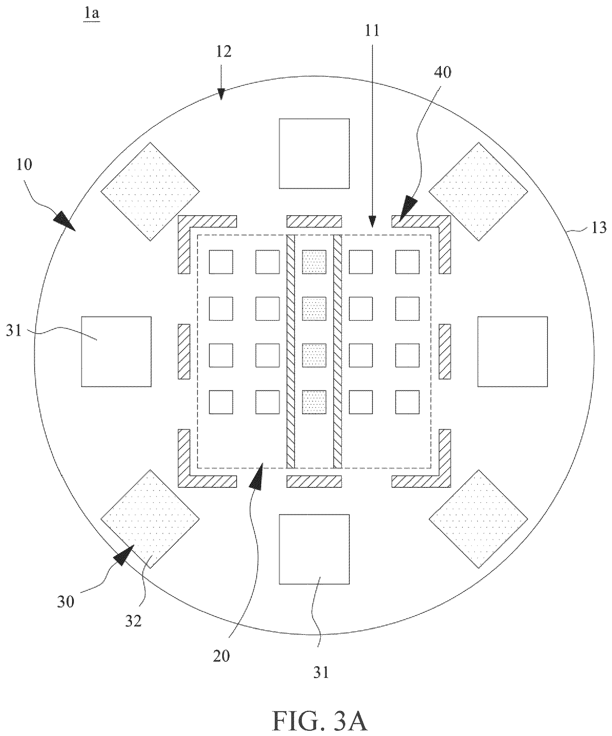

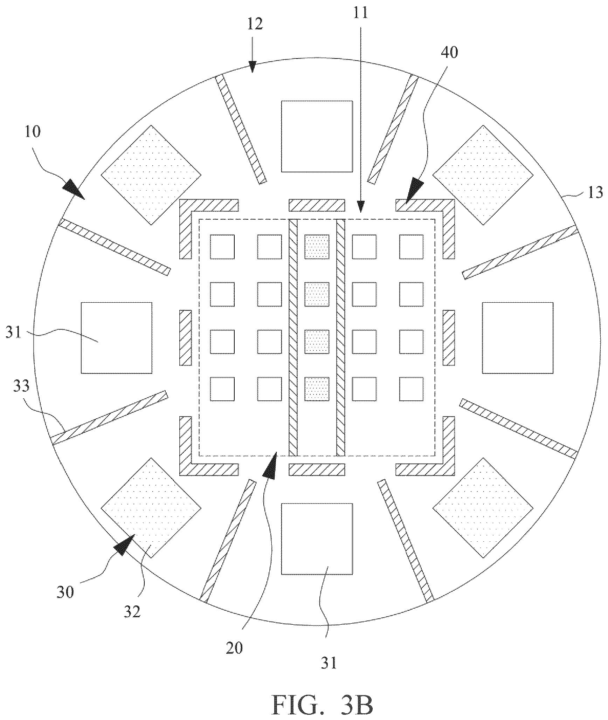

Please refer to FIG. 3A and FIG. 3B together. FIG. 3A is a top view of a second embodiment of the non-invasive blood glucose monitoring device with the wearing fit detection function according to the present invention. FIG. 3B is a top view of at least one blocking structure added for an optical feedback sensing module of the second embodiment shown in FIG. 3A. Compared with the aforementioned first embodiment, this embodiment mainly modifies the shape of the substrate and the disposing positions of the light-emitting elements and the light-receiving elements of the optical feedback sensing module. As shown in FIG. 3A, in this embodiment, a substrate 10 of a non-invasive blood glucose monitoring device with a wearing fit detection function 1a of the present invention is structurally circular, a first setting area 11 is located in a center of a substrate 10, and a second setting area 12 forms an annular area surrounding the first setting area 11. An optical feedback sensing module 30 includes four light-receiving elements 32 and four light-emitting elements 31. The four light-receiving elements 32 are arranged at intervals of 90 degrees at a central angle in the annular area formed by the second setting area 12, and the four light-emitting elements 31 are respectively located in the middle of two adjacent light-receiving elements 32. That is, any light-emitting element 31 and its adjacent light-receiving element 32, are separated by a central angle of forty-five degrees, so that the light-emitting elements 31 and the light-receiving elements 32 are arranged symmetrically relative to a blood glucose monitoring module 20. Also as shown in FIG. 3B, for the second embodiment of the present invention, each blocking structure 33 is further provided between any light-emitting element 31 and its adjacent light-receiving element 32, and each blocking structure 33 extends from an edge 13 of the substrate 10 and approaches any light-blocking wall 40.

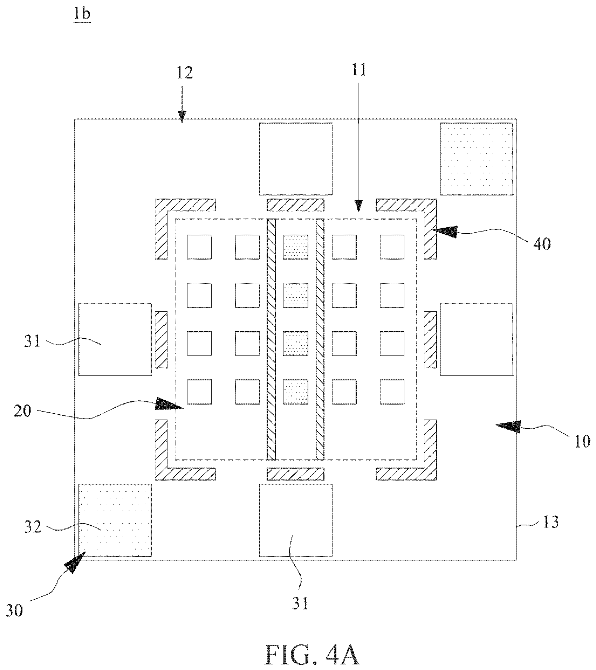

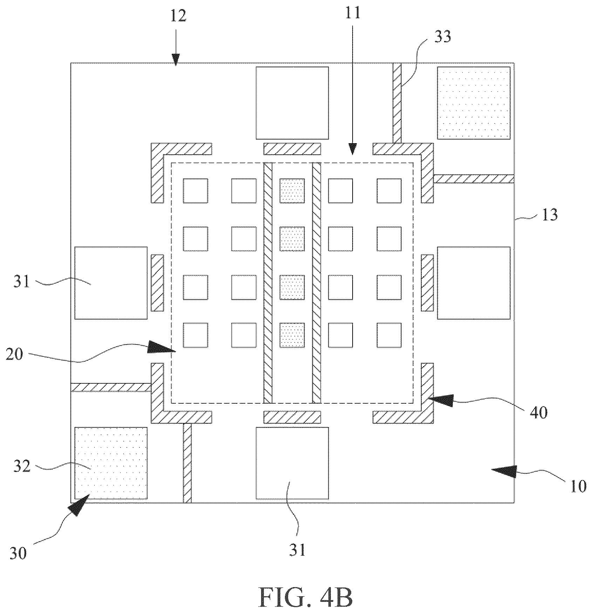

Please refer to FIG. 4A and FIG. 4B together. FIG. 4A is a top view of a third embodiment of the non-invasive blood glucose monitoring device with the wearing fit detection function according to the present invention. FIG. 4B is a top view of at least one blocking structure added for an optical feedback sensing module of the third embodiment shown in FIG. 4A. Compared with the aforementioned first embodiment, this embodiment mainly reduces the quantity of light-receiving elements of the optical feedback sensing module. As shown in FIG. 4A, in this embodiment, an optical feedback sensing module 30 of a non-invasive blood glucose monitoring device with a wearing fit detection function 1b of the present invention includes two light-receiving elements 32 and four light-emitting elements 31. The two light-receiving elements 32 are respectively arranged at two diagonal positions of a rectangular frame area formed by a second setting area 12, while the four light-emitting elements 31 maintain their original positions (as in the first embodiment), which also makes the light-emitting elements 31 and the light-receiving elements 32 arranged symmetrically relative to a diagonal line of a blood glucose monitoring module 20. Accordingly, a detection combination of one light-emitting element 31 and one light-receiving element 32 in each linear portion of the second setting area 12 is formed for the optical feedback sensing module 30. Furthermore, through symmetrically arranging of the two light-receiving elements 32 and the four light-emitting element 31, a wearing fit of the device in whole can be detected. As shown in FIG. 4B, for the third embodiment of the present invention, each blocking structure 33 is further provided between any light-emitting element 31 and its adjacent light-receiving element 32, and each blocking structure 33 extends from an edge 13 of a substrate 10 and connects to any light-blocking wall 40.

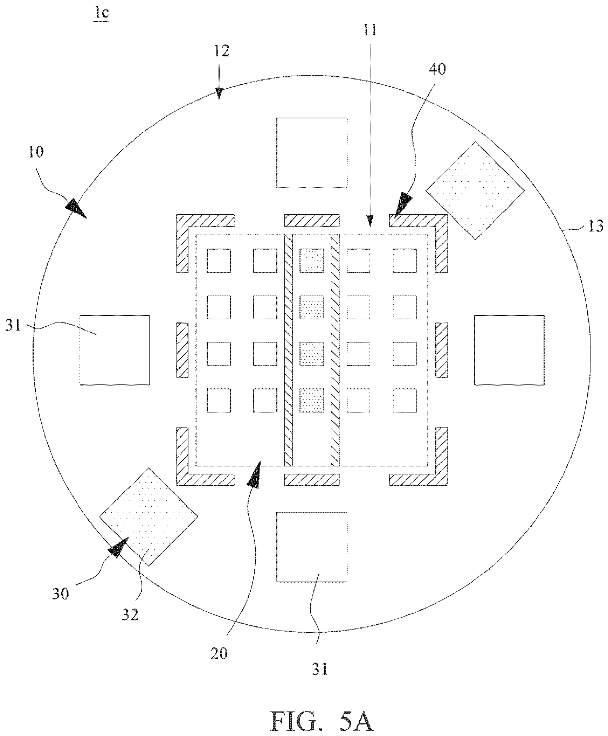

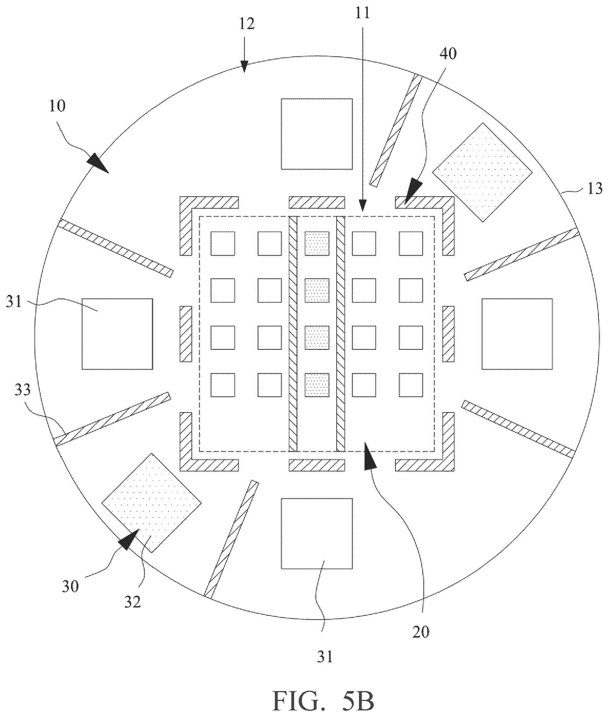

Please refer to FIG. 5A and FIG. 5B together. FIG. 5A is a top view of a fourth embodiment of the non-invasive blood glucose monitoring device with the wearing fit detection function according to the present invention. FIG. 5B is a top view of at least one blocking structure added for an optical feedback sensing module of the fourth embodiment shown in FIG. 5A. Compared with the aforementioned second embodiment, this embodiment mainly reduces the quantity of light-receiving elements of the optical feedback sensing module. As shown in FIG. 5A, in this embodiment, an optical feedback sensing module 30 of a non-invasive blood glucose monitoring device with a wearing fit detection function 1c includes two light-receiving elements 32 and four light-emitting elements 31. The two light-receiving elements 32 are only disposed at intervals of 180 degrees at a central angle in an annular area formed by a second setting area 12, while the four light-emitting elements 31 maintain their original positions (as in the second embodiment), which also makes the light-emitting elements 31 and the light-receiving elements 32 arranged symmetrically relative to a diagonal line of a blood glucose monitoring module 20. Also as shown in FIG. 5B, for the fourth embodiment of the present invention, each blocking structure 33 is further provided between any light-emitting element 31 and its adjacent light-receiving element 32, and each blocking structure 33 extends from an edge 13 of a substrate 10 and approaches any light-blocking wall 40.

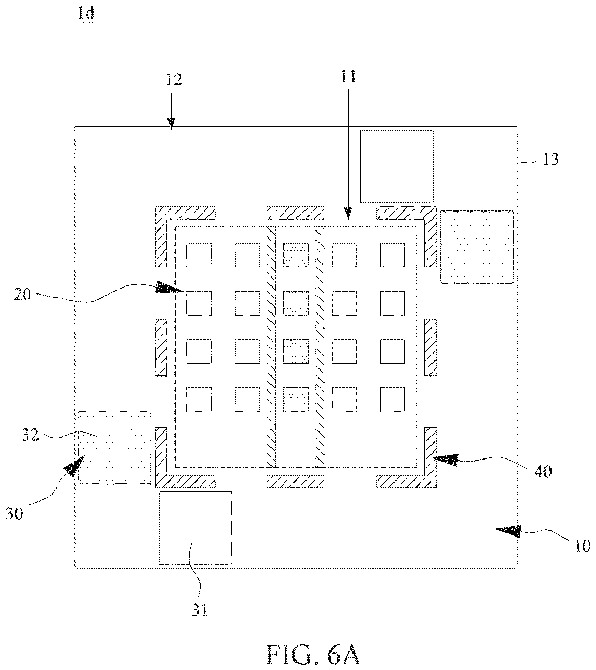

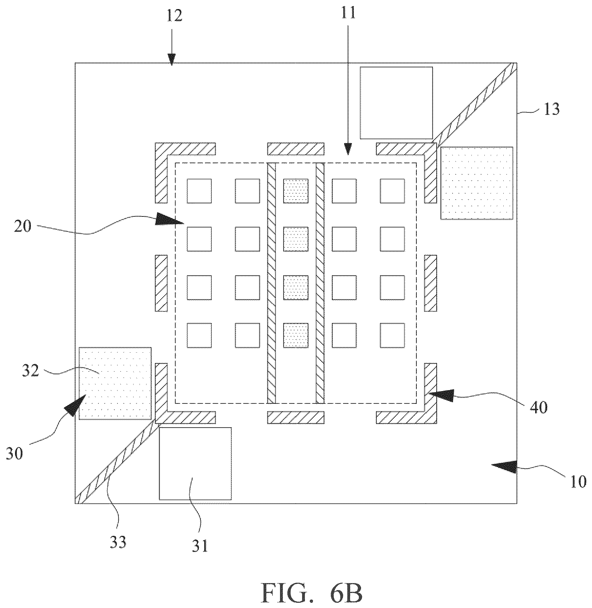

Please refer to FIG. 6A and FIG. 6B together. FIG. 6A is a top view of a fifth embodiment of the non-invasive blood glucose monitoring device with the wearing fit detection function according to the present invention. FIG. 6B is a top view of at least one blocking structure added for an optical feedback sensing module of the fifth embodiment shown in FIG. 6A. Compared with the aforementioned first embodiment, this embodiment mainly reduces the quantity and modifies the disposing locations of the light-emitting elements and the light-receiving elements of the optical feedback sensing module. As shown in FIG. 6A, in this embodiment, an optical feedback sensing module 30 of a non-invasive blood glucose monitoring device with a wearing fit detection function 1d includes two light-receiving elements 32 and two light emitting elements 31. One light-receiving element 32 and one light-emitting component 31 are respectively disposed on both sides of a corner position of a rectangular frame area formed by a second setting area 12, and the other one light-receiving component 32 and the other one light-emitting component 31 are respectively arranged on both sides of a corner position diagonally corresponding to the aforementioned corner position. Accordingly, in each of the two diagonal corner positions of the second setting area 12, a detection combination of one light-emitting element 31 and one light-receiving element 32 is respectively formed for the optical feedback sensing module 30. Moreover, through the two light-receiving elements 32 and the two light-emitting elements 31, a wearing fit of the device in whole is determined. Also as shown in FIG. 6B, for the fifth embodiment of the present invention, each blocking structure 33 is further provided between the light-emitting element 31 and its adjacent light-receiving element 32 of either of the corner positions, and each blocking structure 33 extends from an edge 13 of a substrate 10 and connects to any light-blocking wall 40.

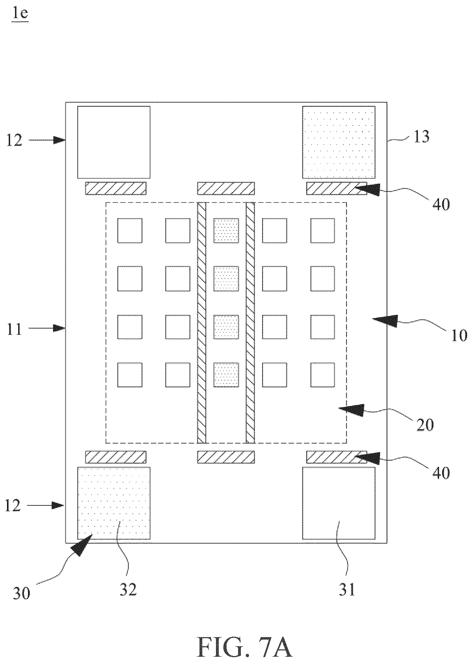

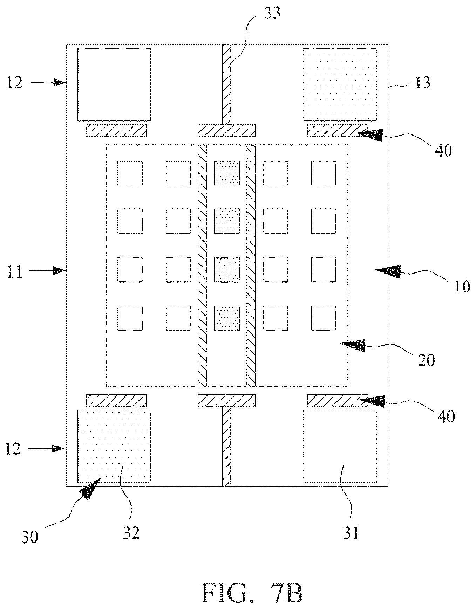

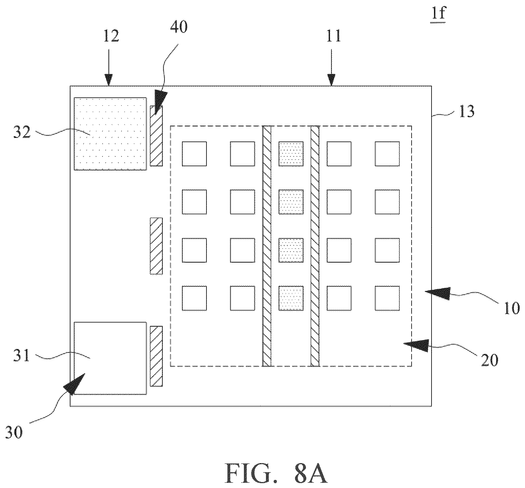

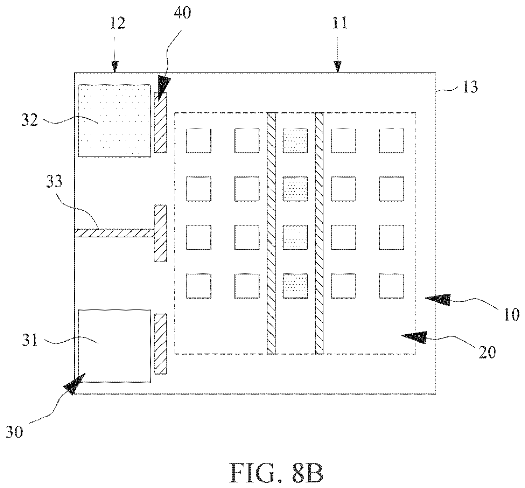

Please refer to FIG. 7A and FIG. 7B together. FIG. 7A is a top view of a sixth embodiment of the non-invasive blood glucose monitoring device with the wearing fit detection function according to the present invention. FIG. 7B is a top view of at least one blocking structure added for an optical feedback sensing module of the sixth embodiment shown in FIG. 7A. Compared with the aforementioned first embodiment, this embodiment mainly modifies the shape and the size of the substrate and the quantity and the disposing positions of the light-emitting elements and the light-receiving elements of the optical feedback sensing module. As shown in FIG. 7A, in this embodiment, a substrate 10 of a non-invasive blood glucose monitoring device with a wearing fit detection function le of the present invention is structurally rectangular, a first setting area 11 is located in a center of the substrate 10, and a second setting area 12 forms a rectangular area located around the first setting area 11. An optical feedback sensing module 30 includes two light-receiving elements 32 and two light-emitting elements 31. One of the light-receiving elements 32 and one of the light-emitting elements 31 are arranged on one side of the rectangular area formed by the second setting area 12, and the other light-receiving elements 32 and the other light-emitting elements 31 are arranged on the other side of the rectangular area formed by the second setting area 12. Accordingly, a detection combination of one light-emitting element 31 and one light-receiving element 32 is formed in each side of the second setting area 12 for the optical feedback sensing module 30. Furthermore, through the two light-receiving elements 32 and the two light-emitting elements 31, a wearing fit of both sides of the device can be determined. Also as shown in FIG. 7B, for the sixth embodiment of the present invention, each blocking structure 33 is further provided between any light-emitting element 31 and its adjacent light-receiving element 32, and each blocking structure 33 extends from an edge 13 of the substrate 10 and connects to any light-blocking wall 40. Please refer to FIG. 8A and FIG. 8B together. FIG. 8A is a top view of a seventh embodiment of the non-invasive blood glucose monitoring device with the wearing fit detection function according to the present invention. FIG. 8B is a top view of at least one blocking structure added for an optical feedback sensing module of the seventh embodiment shown in FIG. 8A. Compared with the aforementioned first embodiment, this embodiment mainly modifies the shape and the size of the substrate and the quantity and the disposing positions of the light-emitting elements and the light-receiving elements of the optical feedback sensing module. As shown in FIG. 8A, in this embodiment, a substrate 10 of a non-invasive blood glucose monitoring device with a wearing fit detection function 1f of the present invention is structurally rectangular, a first setting area 11 is located close to one edge 13 of the substrate 10, and a second setting area 12 forms a rectangular area located outside the first setting area 11 and close to a correspondingly opposite edge 13. An optical feedback sensing module 30 includes one light-receiving element 32 and one light-emitting element 31, and the light-receiving element 32 and the light-emitting element 31 are separately arranged in the rectangular area formed by the second setting area 12. Accordingly, a detection combination of one light-emitting element 31 and one light-receiving element 32 is formed in the second setting area 12 for the optical feedback sensing module 30, to determine a wearing fit of one side of the device. Also as shown in FIG. 8B, for the seventh embodiment of the present invention, a blocking structure 33 is further provided between the light-emitting element 31 and the light-receiving element 32, and the blocking structure 33 extends from the edge 13 of the substrate 10 and connects to any light-blocking wall 40.

The above implementations are only auxiliary descriptions, and are not intended to limit the embodiments of the application subject or the applications or uses of the embodiments. In addition, although at least one illustrative example has been presented above, it should be understood that the present invention can still have a large quantity of variations. It should also be understood that the embodiments described herein are not intended to limit the scope, use, or configuration of the requested subject matter in any way. On the contrary, the foregoing embodiments will provide a convenient guide for those skilled in the art to implement one or more embodiments. Furthermore, various changes can be made to the function and arrangement of the components without departing from the scope defined by the patent claims.

## Claims

1. A non-invasive blood glucose monitoring device with a wearing fit detection function (1), comprising:
a substrate (10), defining a first setting area (11) and a second setting area (12), wherein the second setting area (12) is between an edge (13) of the substrate (10) and the first setting area (11),
a blood glucose monitoring module (20), disposed in the first setting area (11) and including at least one light source (21), at least one photo detector (22) and at least one blocking element (23), wherein the at least one blocking element (23) is utilized to separate the at least one light source (21) and the at least one photo detector (22),
an optical feedback sensing module (30), disposed in the second setting area (12) and including at least one light-emitting element (31) and at least one light-receiving element (32), wherein each light-emitting element (31) emits a light signal corresponding to at least one specific wavelength and each light-receiving element (32) receives the light signal corresponding to the at least one specific wavelength, after being reflected, as a basis to determine a wearing fit, and
at least one light-blocking wall (40), disposed on the substrate (10) and located between the first setting area (11) and the second setting area (12) to block the blood glucose monitoring module (20) and the optical feedback sensing module (30).

2. The non-invasive blood glucose monitoring device with the wearing fit detection function (1) as claimed in the previous claim, wherein each light-emitting element (31) is a multi-chip LED which correspondingly emits a plurality of light signals of specific wavelengths.

3. The non-invasive blood glucose monitoring device with the wearing fit detection function (1) as claimed in the previous claim, wherein the light-receiving element (32) corresponds to a wavelength response range, and the plurality of specific wavelengths fall within the wavelength response range.

4. The non-invasive blood glucose monitoring device with the wearing fit detection function (1) as claimed in the previous claim, wherein the wavelength response range is between 500 nm and 1100 nm.

5. The non-invasive blood glucose monitoring device with the wearing fit detection function (1) as claimed in any of the previous claims, wherein a center distance between each light-emitting element (31) and its adjacent light-receiving element (32) is between 1 mm and 4 mm.

6. The non-invasive blood glucose monitoring device with the wearing fit detection function (1) as claimed in any of the previous claims, wherein a quantity of the at least one light-emitting element (31) is not less than a quantity of the at least one light-receiving element (32).

7. The non-invasive blood glucose monitoring device with the wearing fit detection function (1) as claimed in any of the previous claims, wherein when the at least one light-emitting element (31) and the at least one light-receiving element (32) are both plural, the light-emitting elements (31) and the light-receiving elements (32) are arranged symmetrically relative to the blood glucose monitoring module (20).

8. The non-invasive blood glucose monitoring device with the wearing fit detection function (1) as claimed in any of the previous claims, further comprising a processing unit (70), which mainly adopts photoplethysmography to analyze the reflected light signal corresponding to the at least one specific wavelength for determining the wearing fit.

9. The non-invasive blood glucose monitoring device with the wearing fit detection function (1) as claimed in any of the previous claims, wherein the first setting area (11) is located at a center of the substrate (10), and the second setting area (12) surrounds the first setting area (11).

10. The non-invasive blood glucose monitoring device with the wearing fit detection function (1) as claimed in any of the previous claims, wherein the optical feedback sensing module (30) further includes at least one blocking structure (33), wherein each blocking structure (33) is disposed between any light-emitting element (31) and its adjacent light-receiving element (32), and wherein each blocking structure (33) extends from the edge (13) of the substrate (10) and connects to or approaches any light-blocking wall (40).

11. The non-invasive blood glucose monitoring device with the wearing fit detection function (1) as claimed in any of the previous claims, further comprising a packaging structure (50), disposed on the substrate (10) and adopted to package the blood glucose monitoring module (20) and the optical feedback sensing module (30).

12. The non-invasive blood glucose monitoring device with the wearing fit detection function (1) as claimed in the previous claim, further comprising a transparent cover (60), disposed on the packaging structure (50) to protect the blood glucose monitoring module (20) and the optical feedback sensing module (30).

## Patentansprüche

1. Nichtinvasive Blutzuckerüberwachungsvorrichtung mit einer Funktion zum Erfassen einer Tragepassform (1), aufweisend:
ein Substrat (10), das einen ersten Einstellbereich (11) und einen zweiten Einstellbereich (12) definiert, wobei sich der zweite Einstellbereich (12) zwischen einem Rand (13) des Substrats (10) und dem ersten Einstellbereich (11) befindet;
einem Blutzuckerüberwachungsmodul (20), das im ersten Einstellbereich (11) angeordnet ist und mindestens eine Lichtquelle (21), mindestens einen Fotodetektor (22) sowie mindestens ein Blockierelement (23) aufweist, wobei das mindestens eine Blockierelement (23) dazu verwendet wird, die mindestens eine Lichtquelle (21) und den mindestens einen Fotodetektor (22) voneinander zu trennen;
ein optisches Rückkopplungserfassungsmodul (30), das im zweiten Einstellbereich (12) angeordnet ist und mindestens ein lichtemittierendes Element (31) und mindestens ein Lichtempfangselement (32) aufweist, wobei jedes lichtemittierende Element (31) ein Lichtsignal emittiert, das mindestens einer spezifischen Wellenlänge entspricht, und jedes Lichtempfangselement (32) das der mindestens einen spezifischen Wellenlänge entsprechende Lichtsignal, nachdem es reflektiert wurde, als eine Basis zum Bestimmen einer Tragepassform empfängt; und
mindestens eine lichtblockierende Wand (40), die auf dem Substrat (10) zwischen dem ersten Einstellbereich (11) und dem zweiten Einstellbereich (12) angeordnet ist, um das Blutzuckerüberwachungsmodul (20) und das optische Rückkopplungserfassungsmodul (30) zu blockieren.

2. Nichtinvasive Blutzuckerüberwachungsvorrichtung nach dem vorhergehenden Anspruch, wobei jedes lichtemittierende Element (31) eine Multi-Chip-LED ist, die entsprechend eine Vielzahl von Lichtsignalen spezifischer Wellenlängen emittiert.

3. Nichtinvasive Blutzuckerüberwachungsvorrichtung nach dem vorhergehenden Anspruch, wobei das Lichtempfangselement (32) einem Wellenlängenempfindlichkeitsbereich entspricht und die Vielzahl von spezifischen Wellenlängen innerhalb des Wellenlängenempfindlichkeitsbereichs liegen.

4. Nichtinvasive Blutzuckerüberwachungsvorrichtung nach dem vorhergehenden Anspruch, wobei der Wellenlängenempfindlichkeitsbereich zwischen 500 nm und 1100 nm liegt.

5. Nichtinvasive Blutzuckerüberwachungsvorrichtung nach einem der vorhergehenden Ansprüche, wobei der Mittenabstand zwischen jedem lichtemittierenden Element (31) und seinem benachbarten Lichtempfangselement (32) zwischen 1 mm und 4 mm beträgt.

6. Nichtinvasive Blutzuckerüberwachungsvorrichtung nach einem der vorhergehenden Ansprüche, wobei die Anzahl des mindestens einen lichtemittierenden Elements (31) nicht geringer ist als die Anzahl des mindestens einen Lichtempfangselements (32).

7. Nichtinvasive Blutzuckerüberwachungsvorrichtung nach einem der vorhergehenden Ansprüche, wobei, wenn das mindestens eine lichtemittierende Element (31) und das mindestens eine Lichtempfangselement (32) beide in einer Vielzahl vorliegen, die lichtemittierenden Elemente (31) und die Lichtempfangselemente (32) relativ zum Blutzuckerüberwachungsmodul (20) symmetrisch angeordnet sind.

8. Nichtinvasive Blutzuckerüberwachungsvorrichtung nach einem der vorhergehenden Ansprüche, ferner aufweisend eine Verarbeitungseinheit (70), die hauptsächlich Photoplethysmographie nutzt, um das reflektierte Lichtsignal, das der mindestens einen spezifischen Wellenlänge entspricht, zum Bestimmen der Tragepassform zu analysieren.

9. Nichtinvasive Blutzuckerüberwachungsvorrichtung nach einem der vorhergehenden Ansprüche, wobei sich der erste Einstellbereich (11) in der Mitte des Substrats (10) befindet und der zweite Einstellbereich (12) den ersten Einstellbereich (11) umgibt.

10. Nichtinvasive Blutzuckerüberwachungsvorrichtung nach einem der vorhergehenden Ansprüche, wobei das optische Rückkopplungserfassungsmodul (30) ferner mindestens eine Blockierstruktur (33) aufweist, wobei jede Blockierstruktur (33) zwischen einem beliebigen lichtemittierenden Element (31) und seinem benachbarten Lichtempfangselement (32) angeordnet ist, und wobei sich jede Blockierstruktur (33) vom Rand (13) des Substrats (10) aus erstreckt und mit einer beliebigen lichtblockierenden Wand (40) verbunden ist oder sich dieser annähert.

11. Nichtinvasive Blutzuckerüberwachungsvorrichtung nach einem der vorhergehenden Ansprüche, ferner aufweisend eine auf dem Substrat (10) angeordnete Einkapselungsstruktur (50), die dazu eingerichtet ist, das Blutzuckermessmodul (20) und das optische Rückkopplungserfassungsmodul (30) einzukapseln.

12. Nichtinvasive Blutzuckerüberwachungsvorrichtung nach dem vorhergehenden Anspruch, ferner aufweisend eine transparente Abdeckung (60), die auf der Einkapselungsstruktur (50) angeordnet ist, um das Blutzuckermessmodul (20) und das optische Rückkopplungserfassungsmodul (30) zu schützen.

## Revendications

1. Dispositif de suivi de glycémie non invasif avec une fonction de détection d'ajustement de portage (1), comprenant :
un substrat (10), définissant une première zone d'installation (11) et une seconde zone d'installation (12), dans lequel la seconde zone d'installation (12) est située entre un bord (13) du substrat (10) et la première zone d'installation (11),
un module de suivi de glycémie (20), disposé dans la première zone d'installation (11) et incluant au moins une source lumineuse (21), au moins un photodétecteur (22) et au moins un élément bloquant (23), dans lequel l'au moins un élément bloquant(23) est utilisé pour séparer l'au moins une source lumineuse (21) et l'au moins photodétecteur (22),
un module de détection de rétroaction optique (30), disposé dans la seconde zone d'installation (12) et incluant au moins un élément photoémetteur (31) et au moins un élément photorécepteur (32), dans lequel chaque élément photoémetteur (31) émet un signal lumineux correspondant à au moins une longueur d'onde spécifique et chaque élément photorécepteur (32) reçoit le signal lumineux correspondant à l'au moins une longueur d'onde spécifique, après avoir été réfléchi, en tant que base pour déterminer un ajustement de portage, et
au moins une paroi bloquant la lumière (40), disposée sur le substrat (10) et située entre la première zone d'installation (11) et la seconde zone d'installation (12) pour bloquer le module de suivi de glycémie (20) et le module de détection de rétroaction optique (30).

2. Dispositif de suivi de glycémie non invasif avec la fonction de détection d'ajustement de portage (1) selon la revendication précédente, dans lequel chaque élément photoémetteur (31) est une LED multipuce qui émet de manière correspondante une pluralité de signaux lumineux de longueur d'onde spécifique.

3. Dispositif de suivi de glycémie non invasif avec la fonction de détection d'ajustement de portage (1) selon la revendication précédente, dans lequel l'élément photorécepteur (32) correspond à une plage de réponse en longueur d'onde, et la pluralité de longueurs d'onde spécifiques sont incluses au sein de la plage de réponse en longueur d'onde.

4. Dispositif de suivi de glycémie non invasif avec la fonction de détection d'ajustement de portage (1) selon la revendication précédente, dans lequel la plage de réponse en longueur d'onde est entre 500 nm et 1100 nm.

5. Dispositif de suivi de glycémie non invasif avec la fonction de détection d'ajustement de portage (1) selon l'une quelconque des revendications précédentes, dans lequel une distance entre les centres de chaque élément photoémetteur (31) et de son élément photorécepteur (32) adjacent est entre 1 mm et 4 mm.

6. Dispositif de suivi de glycémie non invasif avec la fonction de détection d'ajustement de portage (1) selon l'une quelconque des revendications précédentes, dans lequel une quantité de l'au moins un élément photoémetteur (31) n'est pas inférieure à une quantité de l'au moins un élément photorécepteur (32).

7. Dispositif de suivi de glycémie non invasif avec la fonction de détection d'ajustement de portage (1) selon l'une quelconque des revendications précédentes, dans lequel lorsque l'au moins un élément photoémetteur (31) et l'au moins un élément photorécepteur (32) sont tous deux plusieurs, les éléments photoémetteurs (31) et les éléments photorécepteurs (32) sont agencés de manière symétrique par rapport au module de suivi de glycémie (20).

8. Dispositif de suivi de glycémie non invasif avec la fonction de détection d'ajustement de portage (1) selon l'une quelconque des revendications précédentes, comprenant en outre une unité de traitement (70), qui adopte principalement la photopléthysmographie pour analyser le signal lumineux réfléchi correspondant à l'au moins une longueur d'onde spécifique pour déterminer l'ajustement de portage.

9. Dispositif de suivi de glycémie non invasif avec la fonction de détection d'ajustement de portage (1) selon l'une quelconque des revendications précédentes, dans lequel la première zone d'installation (11) est située au niveau d'un centre du substrat (10), et la seconde zone d'installation (12) entoure la première zone d'installation (11).

10. Dispositif de suivi de glycémie non invasif avec la fonction de détection d'ajustement de portage (1) selon l'une quelconque des revendications précédentes, dans lequel le module de détection de rétroaction optique (30) inclut en outre au moins une structure bloquante (33), dans lequel chaque structure bloquante (33) est disposée entre n'importe quel élément photoémetteur (31) et son élément photorécepteur (32) adjacent, et dans lequel chaque structure bloquante (33) s'étend depuis le bord (13) du substrat (10) et est reliée à ou s'approche d'une quelconque paroi bloquant la lumière (40).

11. Dispositif de suivi de glycémie non invasif avec la fonction de détection d'ajustement de portage (1) selon l'une quelconque des revendications précédentes, comprenant en outre une structure d'emballage (50), disposée sur le substrat (10) et adoptée pour emballer le module de suivi de glycémie (20) et le module de détection de rétroaction optique (30).

12. Dispositif de suivi de glycémie non invasif avec la fonction de détection d'ajustement de portage (1) selon la revendication précédente, comprenant en outre un couvercle transparent (60), disposé sur la structure d'emballage (50) pour protéger le module de suivi de glycémie (20) et le module de détection de rétroaction optique (30).
